# FASCICULE DE BREVET EUROPEEN

(11) **EP 1 169 983 B1**
(45) Date de publication et mention de la délivrance du brevet: **27.10.2004**
(21) Numéro de dépôt: 01420144.6
(22) Date de dépôt: 27.06.2001
(51) Int. Cl.: A61F 5/01

(54) **Attelle pour articulation humaine avec liberté de pivotement réglable**
Menschliche Gelenkorthese mit verstellbarer Drehachse
Human joint brace having an adjustable range of pivot

(30) Priorité: 07.07.2000 FR 0009071
(43) Date de publication de la demande: 09.01.2002
(73) Titulaire: RICHARD FRERES S.A., 42530 Saint Genest Lerpt (FR)
(72) Inventeur: Richard, Dominique, 42160 Bonson (FR); Voute, Joel, 42230 Roche la Molière (FR)
(74) Mandataire: Maureau, Philippe

(56) Documents cités:
- EP-A- 0 522 484
- WO-A-88/04542
- US-A- 4 489 718
- US-A- 6 080 123

## Description

L'invention est relative à une attelle pour articulation humaine, par exemple pour le genou ou pour le coude, et dont la liberté de pivotement est réglable.

De telles attelles sont utilisées pour compenser des déficiences ligamentaires, ou pour rééduquer des ligaments, de manière progressive, après une opération chirurgicale.

Le document WO88/04542 décrit une attelle de genou dans laquelle deux manchons, enveloppant chacun l'un des deux membres articulés, sont chacun solidaire de deux bras latéraux, respectivement interne et externe, chacun de ces bras étant relié à celui du manchon opposé par une articulation dite flottante. Cette articulation est composée de deux platines en vis à vis, montées pivotantes l'une par rapport à l'autre. Dans une forme d'exécution, chaque articulation comprend une bille d'acier disposée, pour partie dans une rainure rectiligne de l'une des platines, et pour l'autre partie dans une rainure rectiligne de l'autre platine, chacune des rainures ayant son axe longitudinal sensiblement perpendiculaire à l'axe longitudinal du membre dont elle est solidaire. De tels moyens participent au déplacement de l'axe de rotation de l'articulation, de manière à compenser les variations du rayon de courbure du condyle, lorsque la jambe passe d'une position d'extension à une position de flexion, ou inversement, mais n'assurent aucune fonction de limitation de la course. En effet, cette fonction est réalisée par des moyens mécaniques de butée qui ne sont pas réglables en utilisation.

La présente invention a pour objet de fournir une attelle articulée dans laquelle chacune de ses articulations est réglable en utilisation tant en amplitude qu'en position de départ et de fin du pivotement libre, et cela entre une valeur nulle et une valeur maximale. Une attelle selon le préambule de la revendication 1 est connue de US-A-4 489 718.

L'attelle selon l'invention comprend les caractéristiques selon la revendication 1.

En position de fonctionnement, les extrémités libres des arrêts pénétrant dans la rainure circulaire forment donc, dans chacune de ses branches, un obstacle s'opposant au passage des billes dans les deux sens. Ces deux arrêts définissent :
- entre eux et dans la rainure circulaire, un couloir circulaire de réglage,
- et, à partir de chacun d'eux et avec chacune des branches restantes de la rainure circulaire et la rainure longitudinale correspondante, un magasin de stockage des billes inutilisées.

Dans le couloir circulaire de réglage, les billes sont réparties en deux séries, respectivement droite et gauche, disposées de part et d'autre du doigt pénétrant transversalement dans la rainure. Lorsque dans chaque série, les billes s'étendent sans espace entre le doigt et l'arrêt correspondant, l'articulation est totalement bloquée, au jeu fonctionnel près entre les billes. L'angle formé entre les axes longitudinaux des deux bras de l'articulation dépend de la quantité de billes de chacune des séries de billes, respectivement, droite et gauche, disposées dans le couloir.

Pour donner une liberté de pivotement à l'articulation, il suffit de commander l'eclipsage de l'un des arrêts, et de faire pivoter l'un des bras, par exemple celui solidaire du doigt, de manière que le déplacement de ce doigt chasse la quantité de billes excédentaires dans le magasin de stockage dégagé par l'arrêt. Ce réglage réduit le nombre de billes de l'une des séries de billes dans le couloir de réglage, et tolère donc le déplacement du doigt dans la rainure sur une course réglable à volonté et dépendant du nombre de billes chassées.

Le point de départ et de fin du pivotement libre est réglé en modifiant le nombre de billes restantes de chacune des séries, respectivement droite et gauche, contenues dans le couloir de réglage, c'est-à-dire en modifiant la position angulaire de la première bille de chaque série contre laquelle vient buter le doigt dans sa course de pivotement. Ce réglage est effectué, après eclipsage de l'arrêt correspondant, dans un sens, en utilisant le doigt pour chasser la ou les billes excédentaires dans le couloir magasin, et dans l'autre sens, doigt à l'arrêt, en utilisant la poussée du ressort disposé dans la rainure longitudinale correspondante pour chasser hors du couloir magasin, la ou les billes nécessaires, jusqu'à ce qu'elles viennent buter contre le doigt.

Il ressort de ce qui précède que, par sa structure, cette articulation peut être totalement bloquée, ou avoir une liberté de pivotement réglable, tant en amplitude qu'en position, ce qui permet d'adapter parfaitement l'attelle au besoin de rééducation des ligaments.

Dans une forme d'exécution, chaque arrêt eclipsable est constitué par une palette, montée coulissante dans une rainure rectiligne de la platine, et ladite rainure comporte une extrémité débouchant dans la rainure circulaire et une extrémité débouchant dans un logement ménagé dans la platine, ledit logement contenant un ressort poussant la palette en direction de la rainure circulaire et étant traversé, dans son fond, par une lumière laissant passer et se mouvoir un doigt de commande, solidaire de la palette.

Grâce à cet agencement, les moyens de réglage de l'articulation sont très compacts et n'augmentent pas l'encombrement général de l'articulation.

Avantageusement, la platine comportant les rainures, respectivement circulaires et rectilignes, est munie, sur sa face opposée à celle de laquelle débouchent ces rainures, d'une graduation angulaire coopérant avec un index radial porté par le bras de l'autre platine.

Cet aménagement permet de régler plus aisément la course de pivotement et la position de la plage de pivotement libre.

D'autres caractéristiques et avantages ressortiront de la description qui suit en référence au dessin schématique annexé, représentant une forme d'exécution de l'articulation selon l'invention, dans le cas de son application à une attelle pour genou.
Figure 1 est une vue partielle en perspective de l'attelle, lorsqu'elle est mise en position sur un genou,
Figure 2 est une vue de face en élévation montrant, à échelle agrandie, l'articulation,
Figure 3 est une vue similaire à la figure 2, lorsque la platine, formant couvercle, est enlevée et que l'autre platine est retournée de 180°,
Figures 4 et 5 sont des vues en coupe suivant respectivement IV-IV et V-V de figure 3.

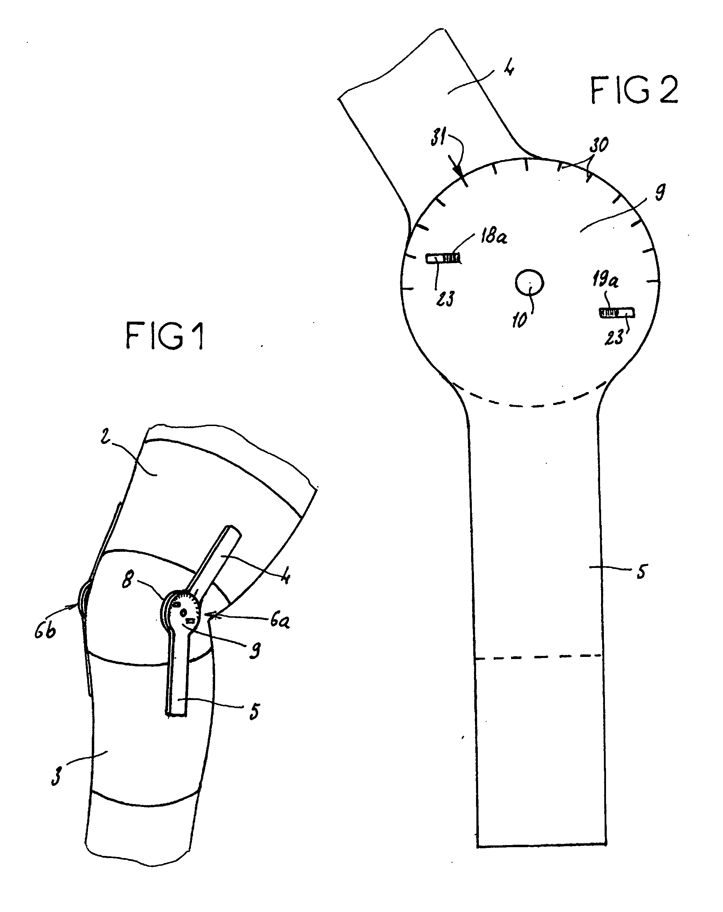

La figure 1 représente une attelle pour articulation du genou composée, de façon connue, de moyens d'enveloppement des membres disposés de part et d'autre de l'articulation dont il faut contrôler le mouvement, et, par exemple, d'un manchon 2 apte à envelopper la cuisse, et d'un manchon 3 apte à envelopper le mollet. Ces deux éléments qui, par exemple, sont liés l'un à l'autre par des sangles ou par des bandes de velours à crochets coopérant avec des bandes de velours à bouclettes, peuvent être remplacés par tout autre moyen permettant d'assurer la liaison des bras 4 et 5, faisant partie de chacune des deux articulations 6a, et 6b, disposées de part et d'autre du genou. Chaque bras 4 et 5 est solidaire d'une platine, respectivement 8 et 9, au moins partiellement circulaire. Chacun des bras 4 et 5 est lié fermement au manchon respectif 2 et 3, éventuellement avec l'aide de renforts non représentés, métalliques ou en matière plastique.

Selon l'invention, et comme montré aux figures 2 à 5, les deux platines 8 et 9 sont articulées l'une sur l'autre par un axe traversant 10, représenté ici comme étant un rivet, mais pouvant se présenter sous toute autre forme.

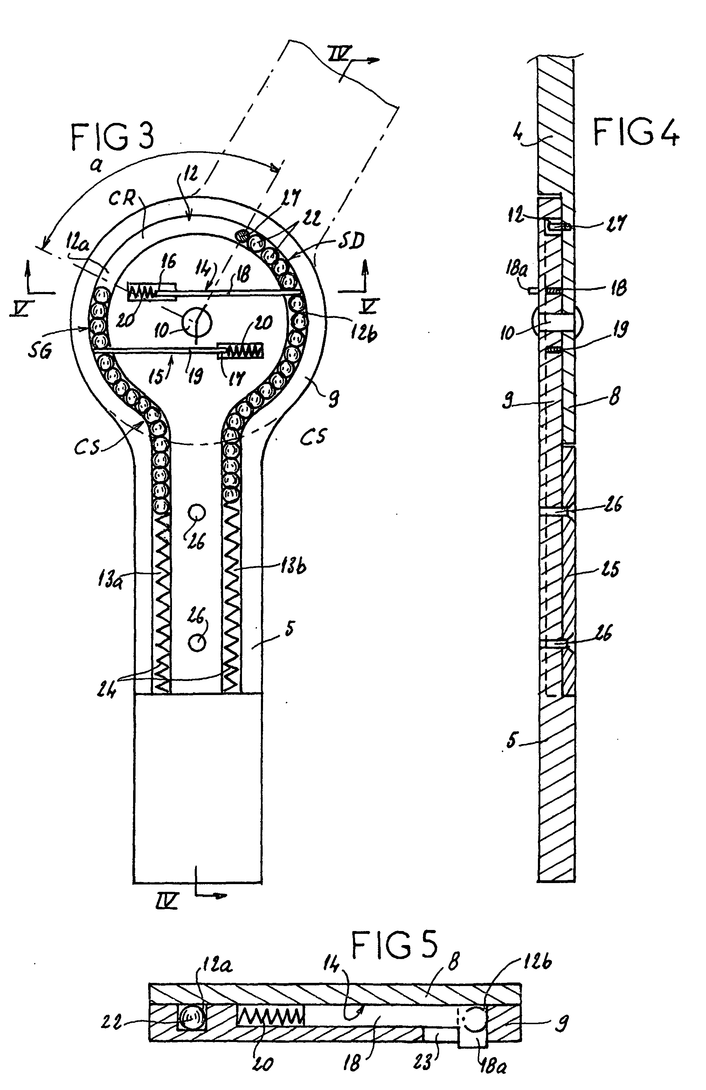

La platine 9 comprend une rainure circulaire 12, qui s'étend sur plus de 180° et en l'occurrence sur 300°, et dont chacune des branches 12a, 12b se raccorde avec l'une des deux rainures longitudinales 13a, 13b ménagées dans le bras 5 correspondant. La platine 9 comprend également deux rainures rectilignes 14 et 15 qui, dans cette forme d'exécution sont parallèles et disposées de part et d'autre de son axe de rotation. Chaque rainure débouche, d'un côté, dans l'une des branches de la rainure circulaire 12, à savoir celle 12b pour la rainure 14 et celle 12a pour la rainure 15, et, de l'autre côté, dans un logement, respectivement 16 et 17. Chacune des rainures 14 et 15 contient une palette respectivement 18 et 19, pouvant coulisser librement dans sa rainure respective 14 et 15. L'une des extrémités de chaque palette prend appui, dans le logement 16 ou 17 correspondant, sur un ressort 20 tendant à la déplacer dans la rainure correspondante 14, 15, de manière que son extrémité libre sécante la branche 12a, 12b de la rainure circulaire, en formant un arrêt, c'est-à-dire un obstacle au déplacement des billes 22 disposées dans cette rainure. Chacune des palettes 18 ou 19 est solidaire d'un doigt 18a, 19a, qui traverse le fond de la platine 9 par une lumière 23 plus longue que lui. Ce doigt forme une saillie dépassant de la face externe de cette platine, et permettant de déplacer manuellement la palette pour l'amener de sa position de travail, représentée aux figures 3 à 5, à une position éclipsée dans laquelle son extrémité libre n'est plus dans la branche correspondante du couloir circulaire.

Dans la forme d'exécution représentée, les deux palettes 18 et 19 sont disposées parallèlement et de part et d'autre de l'axe 10, mais elles peuvent prendre toute autre position, en fonction des besoins du réglage pourvu que chacune d'elles ait une extrémité qui sécante l'une des deux branches 12a, 12b de la rainure circulaire 12.

Les rainures longitudinales 13a, 13b contiennent chacune un ressort hélicoïdal de compression 24, de faible raideur et grande longueur, tendant à chasser les billes 22 en direction de la rainure circulaire 12.

La platine 9 forme un couvercle qui maintient les billes dans la rainure circulaire 12, tandis qu'un couvercle 25 est rapporté sur le bras 5 pour maintenir les billes dans les deux rainures longitudinales 13a, 13b. Ce couvercle 25 est fixé sur le bras 5 par deux vis 26, ou par tout autre moyen équivalent.

Enfin, l'articulation comprend aussi un doigt transversal 27, porté par la platine 8, pénétrant dans la rainure circulaire 12 et pouvant librement se mouvoir dans celle-ci.

Avant d'expliquer le fonctionnement de l'articulation, il est précisé que les deux palettes 15 et 18 divisent le couloir circulaire 12 en deux parties, une partie supérieure dite couloir de réglage CR et une partie inférieure CS comprenant le reste des branches 12a, 12b et leur raccordement aux branches longitudinales 13a, 13b, formant deux couloirs de stockage de la réserve de billes.

Lorsque l'attelle est dans une position avec liberté de pivotement contrôlé, comme montré à la figure 3, le couloir de réglage CR contient une série de billes disposées à gauche SG dans la branche 12a, et une série de billes disposées à droite SD dans la branche 12b. Dans ces conditions, la course libre de pivotement est déterminée par butée du doigt 27 sur la bille supérieure de chacune des séries s'effectue donc sur une valeur angulaire a comme montré à la figure 3. Pour modifier cette valeur angulaire, dans le sens d'une augmentation, il faut réduire le nombre de billes de l'une ou des deux séries de billes SG, SD, tandis que, pour la réduire, il faut augmenter le nombre de billes de l'une et/ou l'autre des deux séries. De même, pour modifier le point de départ et le point d'arrêt de la course, c'est-à-dire la position angluaire de la course au couloir de réglage CR, il faut modifier le nombre de billes dans chacune des deux séries, et par exemple, enlever une bille dans la série gauche SG, et rajouter une bille dans la série droite SD.

Ces différents réglages sont aidés, comme montré figure 2, par une graduation angulaire 30, ménagée sur la face de la platine 9 qui est opposée à celle de laquelle débouchent les rainures 12, 13a et 13b. Cette graduation coopère avec un index 31 ménagé sur le bras 4. Bien entendu, la graduation est en rapport avec la dimension des billes de manière que tout déplacement de l'index 31 entre deux repères de la graduation 30 corresponde à un nombre entier de billes chassées ou acceptées dans le couloir de réglage CR.

Ainsi, pour effectuer le réglage, par exemple de modification de la course angulaire a, dans le sens d'une augmentation, de la valeur de la liberté de flexion de la jambe, il suffit d'agir sur le doigt de commande 18a pour amener la palette 18 en position d'eclipsage, libérer la branche 12a de la rainure circulaire 12, et permettre, par pivotement relatif du bras 4 par rapport au bras 5, de réduire le nombre de billes de la série SD jusqu'à obtention de l'angle désiré. A l'inverse, pour réduire cet angle, avec la palette 18, en position éclipsée, il faut déplacer le bras 4 vers la gauche pour permettre au ressort 13b de pousser une ou plusieurs billes du couloir de stockage dans le couloir de réglage, et précisément, dans la branche 12b.

La mise en position éclipsée de la palette 19 permet, en modifiant le nombre de billes de la série SG, d'augmenter la course ou de modifier la position de fin de course de l'articulation mécanique, par exemple en fin d'extension de jambe.

Il ressort de ce qui précède que ce dispositif, simple et peu encombrant, permet de régler à volonté et en fonction des besoins de rééducation, non seulement l'amplitude du pivotement libre de l'articulation mais aussi la position de ce pivotement par rapport à un élément de référence.

De même, en remplissant le couloir de réglage CR de billes 22, de manière que les deux séries SG, SD, soient au contact du doigt 27, et de chaque côté, il est possible d'assurer l'immobilisation de l'articulation. Cela permet d'utiliser l'attelle immédiatement après une opération ligamentaire et d'augmenter progressivement la liberté de pivotement, sans avoir à se procurer une autre attelle.

## Revendications

1. Attelle pour articulation humaine avec liberté de pivotement réglable comprenant, pour chacun des membres de l'articulation humaine, des moyens (2, 3) d'enveloppement du membre solidaires de deux bras (4 ou 5), respectivement interne et externe, et dans laquelle, d'une part, chacun de ces bras saillant en direction de l'articulation humaine, est lui-même solidaire d'une platine (8, 9) au moins partiellement circulaire, par laquelle il vient en contact avec la platine du bras disposé du même côté, et est articulé autour d'un axe géométrique horizontal et transversal, et, d'autre part, chaque articulation mécanique comprend des moyens de butée limitant la course de pivotement de l'une des platines (8, 9) par rapport à l'autre, et les deux platines (8, 9) de chaque articulation mécanique sont articulées autour d'un axe (10) les traversant, tandis que :
- d'une part, l'une des platines (9) comprend:
a) centrée sur son axe géométrique d'articulation, une rainure (12) s'étendant sur au moins 180° et dont chaque branche (12a, 12b) se raccorde avec l'une des deux rainures longitudinales (13a, 13b), ménagées dans le bras (5) correspondant,
- et que, d'autre part, l'autre platine (8) recouvre la rainure circulaire (12) et est solidaire d'un doigt transversal (27) pénétrant dans cette rainure (12) et pouvant circuler dans celle-ci, **caractérisée en ce que** la platine comprenant la rainure comprend:
a) une rainure circulaire
b) des billes ( 22) disposées dans les rainures,
c) deux ressorts (24). disposés chacun dans l'une des deux rainures longitudinales (13a, 13b) pour pousser les billes vers la rainure circulaire (12),
et d) deux arrêts eclipsables (18, 19) disposés en opposition et présentant une extrémité libre sécantant l'une des branches (12a, 12b) de la rainure circulaire (12) pour former butée de limitation de course,

2. Attelle selon la revendication 1, **caractérisée en ce que** chaque arrêt eclipsable est constitué par une palette (18, 19) montée coulissante dans une rainure rectiligne (14, 15) de la platine (9) , ladite rainure comportant une extrémité débouchant dans la rainure circulaire (12) et une extrémité débouchant dans un logement (16) ménagé dans la platine, ce logement contenant un ressort (24) poussant la palette en direction de la rainure circulaire (12) et étant traversé, dans son fond, par une lumière (23) laissant passer et se mouvoir un doigt de commande (18a, 18b), solidaire de la palette (18, 19).

3. Attelle selon la revendication 1, **caractérisée en ce que** les rainures longitudinales (13a, 13b) du bras sont couvertes par un couvercle (25) rapporté sur ce bras.

4. Attelle selon la revendication 1, **caractérisée en ce que** la platine (9) comportant les rainures, respectivement circulaire (12) et rectilignes (13a, 13b), est munie, sur sa face opposée à celle de laquelle débouchent ces rainures, d'une graduation angulaire (30) coopérant avec un index radial (31) porté par le bras (4) de l'autre platine (8).

## Patentansprüche

1. Schiene für ein menschliches Gelenk mit einstellbarer freier Schwenkbarkeit, welches für jedes der Glieder des menschlichen Gelenks Umhüllungsmittel (2, 3) des Gliedes aufweist, die mit einem inneren und einem äusseren Arm (4 oder 5) verbunden sind, und in welcher einerseits jeder Arm der in der Richtung des menschlichen Gelenks hervorstehenden Arme seinerseit mit einer zumindest teilweise kreisförmigen Platte (8, 9) verbunden ist, mittels derer er mit der Platte des auf derselben Seite angeordneten Armes in Kontakt gelangt, und um eine horizontale und transversale geometrische Achse herum angelenkt ist, und andererseits jedes mechanische Gelenk Anschlagsmittel aufweist, welche den Schwenkhub einer der Platten (8, 9) bezüglich der anderen begrenzt, wobei die beiden Platten (8, 9) jedes mechanischen Gelenks um eine diese durchquerende Achse (10) angelenkt sind, wobei:
- einerseits eine der Platten (9) aufweist:
a) zentriert auf ihrer geometrischen Gelenkachse eine Rille (12), die sich über mindestens 180° erstreckt und bei der jeder Schenkel (12a, 12b) sich mit der einen der beiden Längsrillen (13a, 13b) vereint, die in den entsprechenden Arm (5) eingearbeitet sind,
- und wobei andererseits die andere Platte (8) die kreisförmige Rille (12) verdeckt und mit einem Querfinger (27) verbunden ist, der in diese Rille (12) eindringt und in ihr zirkulieren kann,
**dadurch gekennzeichnet, dass**
die die Rille aufweisende Platte aufweist:
a) eine kreisförmige Rille,
b) Kugeln (22), die in den Rillen angeordnet sind,
c) zwei Federn (24), die jeweils in einer der beiden Längsrillen (13a, 13b) angeordnet sind, um die Kugeln zu der kreisförmigen Rille (12) zu drücken, und
d) zwei versenkbare Sperren (18, 19), die gegenüberliegend angeordnet sind und ein freies Ende haben, das einen der Schenkel (12a, 12b) der kreisförmigen Rille (12) schneidet, um einen Anschlag zur Hubbegrenzung zu bilden.

2. Schiene nach Anspruch 1, **dadurch gekennzeichnet, dass** jede versenkbare Sperre aus einer Palette (18, 19) gebildet ist, die in einer geradlinigen Rille (14, 15) der Platte (9) gleitend gelagert ist, wobei die Rille ein in die kreisförmige Rille (12) mündendes Ende und ein in einen in die Platte eingearbeiteten Sitz (16) mündendes Ende aufweist, wobei dieser Sitz eine Feder (24) enthält, welche die Platte in die Richtung der kreisförmigen Rille (12) schiebt, und der in seinem Boden von einem Längsloch (23) durchquert ist, welches einen mit der Palette (18, 19) verbundenen Steuerfinger (18a, 18b) hindurchtreten lässt und seine Bewegung ermöglicht.

3. Schiene nach Anspruch 1, **dadurch gekennzeichnet, dass** die Längsrillen (13a, 13b) des Arms durch einen an diesem Arm befestigten Deckel (25) abgedeckt sind.

4. Schiene nach Anspruch 1, **dadurch gekennzeichnet, dass** die Platte (9), welche die kreisförmige Rille (12) bzw. die geradlinigen Rillen (13a, 13b) aufweist, auf ihrer Seite, die zu derjenigen entgegengesetzt ist, von denen aus diese Rillen münden, mit einer Winkel-Gradeinteilung (30) ausgestattet ist, die mit einem radialen Finger (31) zusammenwirkt, der von dem Arm (4) der anderen Platte (8) getragen wird.

## Claims

1. Splint for a human articulation with adjustable freedom to pivot comprising, for each of the members of the human articulation, means (2, 3) for enveloping the member secured to two internal or external arms respectively (4 or 5), and wherein, on the one hand each of these arms projecting in the direction of the human articulation, is itself secured to an at least partly circular plate (8, 9), by which it comes into contact with the plate of the arm positioned on the same side, and is articulated about a horizontal transverse geometrical axis and, on the other hand, each mechanical articulation includes stopping means limiting the pivoting travel of one of the plates (8, 9) with respect to the other, and the two plates (8, 9) of each mechanical articulation are articulated about a pivot pin (10) passing through them, while:
- on the one hand, one of the plates (9) includes:
a) centred on its geometrical axis of articulation, a groove (12) extending over at least 180° and of which each branch (12a, 12b) connects with one of the two longitudinal grooves (13a, 13b) provided in the corresponding arm (5),
- and, on the other hand, the other plate (8) covers the circular grove (12) and is secured to a transverse finger (27) entering this groove (12) and that is capable of circulating therein, **characterized in that** the plate including the groove comprises:
a) a circular groove
b) balls (22) positioned in the grooves,
c) two springs (24), each positioned in one of the two longitudinal grooves (13a, 13b) in order to push the balls towards the circular groove (12), and
d) two retractable stops (18, 19) positioned in opposition and having a free end intersecting one of the branches (12a, 12b) of the circular grove (12) so as to form a stop limiting travel.

2. Splint according to claim 1, **characterized in that** each retractable stop consists of a blade (18, 19) mounted slidingly in a rectilinear grove (14, 15) of the plate (9), the said grove having an end emerging in the circular groove (12) and an end emerging in a housing (16) provided in the plate, this housing containing a spring (24) pushing the blade in the direction of the circular grove (12) and being traversed, at its bottom, by an opening (23) allowing a control finger (18a, 18b), secured to the blade (18, 19), to move.

3. Splint according to claim 1, **characterized in that** the longitudinal grooves (13a, 13b) of the arm are covered by a cover (26) added to this arm.

4. Splint according to claim 1, **characterized in that** the plate (9) including circular (12) and rectilinear (13a, 13b) grooves respectively, is provided, on its face opposite that from which these grooves emerge, with an angular graduation (30) cooperating with a radial index (31) carried by the arm (4) of the other plate (8).
